# EUROPEAN PATENT SPECIFICATION

(11) **EP 0 627 216 B1**
(45) Date of publication and mention of the grant of the patent: **06.08.2003**
(21) Application number: 94107636.6
(22) Date of filing: 17.05.1994
(51) Int. Cl.: A61K 7/50, A61K 7/08

(54) **Stable conditioning shampoo containing an anionic surfactant, a fatty alcohol, a silicone conditioner and polyethyleneimine**
Stabiles konditionierendes Haarpflegeshampoo enthaltend ein aminonisches Tensid, eines Fettalkohol, ein Silicone-Konditionierungsmittel und Polyethyleneimine
Shampooing de conditionnement stable contenant une tensioactive anionique, un alcool gras, un conditionneur silicone et polyethyleneimine

(30) Priority: 17.05.1993 US 63015
(43) Date of publication of application: 07.12.1994
(73) Proprietor: UNILEVER PLC, London EC4P 4BQ (GB); UNILEVER N.V., 3013 AL Rotterdam (NL)
(72) Inventor: Pyles, Daniel Raymond, Chicago, Illinois 60630 (US)
(74) Representative: Griffiths, Helen Sarah

(56) References cited:
- FR-A- 2 289 167
- US-A- 4 381 259
- US-A- 5 104 645

## Description

### FIELD OF INVENTION

The present invention is directed to a hair conditioning shampoo composition and to a method of treating hair with the composition to cleanse the hair and, at the same time, to provide the hair with improved wet-stage and dry-stage conditioning properties as well as other conditioning properties, such as softness, without residual buildup of conditioning agents on the hair. More particularly, the present invention is directed to a hair conditioning shampoo composition including one or more anionic cleaning surfactants, such as ammonium lauryl sulfate; one or more non-volatile silicone oils, such as a polydimethylsiloxane compound; a long-chain fatty alcohol, preferably predominantly C₁₂, C₁₄, and/or C₁₆ fatty alcohol(s); and a cationic (protonated) polyethyleneimine. Surprisingly, the combination of one or more long-chain (C₈-C₁₈) fatty alcohols, and a cationic polyethyleneimine effectively increases foam volume and conditioning properties while maintaining excellent cleansing in a composition that is stable over extended periods of time at elevated temperatures.

### BACKGROUND OF THE INVENTION AND PRIOR ART

Soiled human hair is shampooed to remove sebum that is naturally secreted by the head, as well as soil and other atmospheric contaminants that accumulate on the hair. Sebum, in particular, accumulates on the hair in a relatively short period of time leaving the hair with a greasy, dirty feel and poor manageability. The most effective shampoos for cleansing the hair for removal of the atmospheric contaminants and sebum are those that contain high lather synthetic anionic detergents, such as the long-chain alkyl sulfates and the partially ethoxylated long-chain alkyl sulfates. These synthetic anionic detergents are very effective for cleansing the hair but, after rinsing with water, leave the hair with a dried touch, usually called "creak" and result in hair, when wet, that is in an extremely tangled and unmanageable after-shampoo condition.

Thoroughly cleansed hair is extremely difficult to comb in either the wet or dry state because the individual hair fibers tend to snarl, kink, and interlock with each other. Particularly prior to complete drying of thoroughly cleansed hair in this after-shampoo stage the hair is very difficult to comb or brush. Even after complete drying, the thoroughly cleansed hair remains difficult to comb or brush and does not set well. Thoroughly clean, dried hair also has undesirable electrostatic properties in a low humidity atmosphere that causes the hair to "fly away," thereby further reducing the combing or brushing property of the hair. Generally, these above-outlined problems that result from synthetic detergent cleansing of the hair, particularly the high-lather synthetic anionic detergents, have been elevated either by the after-shampoo treatment of the hair with hair conditioners, for example in the form of a hair rinse, or by including hair conditioners directly within the shampoo composition.

After-shampoo hair conditioning compositions are easily formulated but are inconvenient to use because of the necessity of applying the conditioner to the hair in a separate stage after shampooing. The preparation of a conditioning shampoo has been more difficult because of inherent incompatibility problems between anionic surfactants and the fatty cationic compounds that are good conditioning agents. Contact between an anionic surfactant and a cationic surfactant or cationic polymer produces a precipitate that forms immediately or causes an interaction between the anionic and cationic compounds that significantly reduces their respective cleaning and conditioning properties. The reduction in cleansing and conditioning effectiveness is observed even in compositions wherein the anionic and cationic compounds do not precipitate from the composition but remain in solution or suspension. This incompatibility between an anionic surfactant and a cationic conditioning compound is well recognized by those skilled in the art. For example, Safarin in *Cosmetics,* Interscience Publishers, Inc., New York, p. 538 (1957), states that anionic and cationic compounds cannot be used in combination because they react to form insoluble salts.

A partial solution to this incompatibility problem in the formulation of conditioning shampoos is exemplified by the following patents that disclose compositions that contain surfactants that are not anionic, e.g., nonionics, amphoterics and zwitterionics, together with cationic conditioning compounds: U.S. Patent No. 3,849,348 to Hewitt; U.S. Patent No. 3,990,991 to Gerstein; and U.S. Patent No. 3,822,312 to Sato. Published European Patent Application EP 0 407 042 A2 teaches long chain alcohols, long chain ethoxylated alcohols and/or long chain esters or acids in conditioning shampoos.

Another problem inherent in formulating a conditioning shampoo is an instability problem that results when water-insoluble conditioning agents are also included in the conditioning shampoo composition, such as the non-volatile silicones that are well recognized in the art as providing a degree of softness to the hair.

Silicones in shampoo compositions have been disclosed in a number of different patents: U.S. Patent No. 2,826,551, Marsh 11, 1958 to Green; U.S. Patent No. 3,964,500, June 22, 1976 to Drakoff; U.S. Patent No. 4,364,837, December 21, 1982 to Pader; British Patent No. 849,433, September 28, 1960 to Woolston; U.S. Patent No. 4,741,855 to Grote et al.; U.S. Patent Nos. 4,788,006 and 4,902,449 to Bolich, Jr. et al.; and U.S. Patent No. 4,704,272 to Oh et al.

A particularly difficult problem to solve in silicone-containing conditioning shampoos is that of keeping a dispersed, water-insoluble, non-volatile silicone material suspended in stable form while retaining the cleansing, particularly foam volume, and conditioning performance of the conditioning shampoo. A variety of materials have been proposed for inclusion in silicone-containing conditioning shampoos for purposes of thickening and stabilization such as xanthan gum, long-chain acyl derivatives, long-chain amide oxides, and long-chain alkanolamides as disclosed in U.S. Patent Nos. 4,788,006, 4,704,272, and 4,741,855. The compositions of the present invention are stable and have increased foam volume and conditioning properties without amine oxide or acyl derivative suspending agents.

### SUMMARY OF THE INVENTION

In accordance with the principles of the present invention, it has been found, surprisingly, that a conditioning shampoo containing an anionic surfactant, a long-chain fatty alcohol, a cationic polyethyleneimine, and a non-volatile silicone material has extended product stability, excellent cleansing and foaming properties, and provides excellent and improved overall conditioning to human hair, particularly superior wet and dry combing properties.

Hair treated with the composition of the present invention is thoroughly cleansed with increased foam and exhibits improved physical and cosmetic properties, such as gloss, thickness, manageability, softness and body. Further, it was surprisingly and unexpectedly found that hair treated with the composition of the present invention does not experience buildup on the hair shaft, over time, of conditioning agents, as is common with many conditioning shampoo compositions.

Therefore, an aspect of the present invention is to provide a hair-treating composition that cleanses the hair, provides improved foam volume, and imparts improved physical properties and cosmetic properties to the hair in a single application.

Another aspect of the present invention is to provide a physically stable conditioning shampoo containing an anionic surfactant, a cationic (protonated) polyethyleneimine polymer and a non-volatile silicone.

Another aspect of the present invention is to provide a new and improved conditioning shampoo containing a strong anionic detergent, such as a long-chain alkyl sulfate, long-chain alkyl ether sulfate, and/or long-chain sulfonate; a non-volatile silicone conditioning agent; a cationic polyethyleneimine, and a long-chain (C₈-C₁₈) fatty alcohol to provide improved foaming, conditioning, and stability.

Still another aspect of the present invention is to provide a new and improved conditioning shampoo including 5% to 65% of an anionic surfactant; 0.5% to 10% of a non-volatile silicone material; 2% to 10% of a long-chain (predominantly C₈-C₁₈), preferably predominantly C₁₂-C₁₆ fatty alcohol(s); and 0.01% to 4%, preferably .01% to 1%, cationic (protonated) polyethyleneimine, said composition having a pH of 4 to 8.

A further aspect of the present invention is to provide a new and improved method of making an aqueous conditioning shampoo having an anionic surfactant, a cationic polyethyleneimine, a suspended non-volatile silicone, and a fatty alcohol, by vigorously mixing the composition, together with the fatty alcohol, to suspend silicone droplets having a particle size in the range of about 5 microns to about 100 microns to provide new and unexpected stability to the conditioning shampoo composition without sacrifice in foaming.

Still another aspect of the present invention is to provide a new and improved conditioning shampoo having a pH in the range of 4 to 8, preferably 4 to 7, and more preferably 5 to 6 including 5% to 65% of an anionic surfactant; a cationic (protonated) polyethyleneimine in an amount of 0.01% to 4% by weight, preferably 0.01% to 2% by weight, more preferably 0.1% to 1% by weight; and optionally, 0.1% to 20% of a cationic, nitrogen-containing conditioning agent having only two long-chain alkyl radicals bonded to the nitrogen atom, the long-chain radicals having predominantly about 12 to about 22 carbon atoms per long-chain alkyl radical; 0.5% to 10% of a non-volatile silicone material; and 2% to 10% of a long-chain fatty alcohol.

Another aspect of the present invention is to provide a new and improved conditioning shampoo having a pH in the range of 4 to 7, preferably 5 to 6, including 5% to 65% of an anionic surfactant; a cationic protonated polyethyleneimine in an amount of 0.01% to 4%, preferably 0.01% to 1% by weight, and having a cationic polymer charge density of at least about 10 milliequivalents per gram, preferably about 15 to about 20 milliequivalents per gram; optionally, about 0.1% to about 20% of a cationic, nitrogen-containing conditioning agent having only two long-chain alkyl radicals bonded to the nitrogen atom, the long-chain radicals having predominantly 12 to 22 carbon atoms per long-chain alkyl radical; 0.5% to 10% of a non-volatile silicone material; and 2% to 10% of a long-chain fatty alcohol.

The above and other aspects and advantages of the present invention will become apparent from the following detailed description of the preferred embodiments.

### DETAILED DESCRIPTION OF THE PREFERRED EMBODIMENTS

The aqueous conditioning shampoo composition of the present invention generally includes an anionic surfactant in an amount of 5% to 65%, preferably 5% to 25%, by weight of the composition; 0.01% to 4% by weight of a cationic polyethyleneimine; one or more water-insoluble long chain (C₈-C₁₈) fatty alcohols in an amount of 2 % to 20% by weight of the composition; and one or more non-volatile silicone conditioning agents in an amount of 0.5% to 10% by weight of the composition.

The conditioning shampoo of the present invention provides the hair with improved foam, as well as improved physical and cosmetic conditioning properties, such as gloss, thickness, softness, and manageability, including excellent wet and dry combing properties and body. The composition of the present invention remains stable, while achieving excellent cleansing, foaming, and conditioning.

The anionic cleansing surfactant used in the composition and method of the present invention can be any of the anionic surfactants known or previously used in the art of hair shampoos. An anionic cleansing surfactant should be included in the composition of the present invention to effectively cleanse the hair and generates a high, stable foam level that consumers equate with cleaning efficiency. Nonionic and amphoteric surfactants are not as effective in cleansing the hair and do not provide the high foam level desired by consumers. However, optionally, nonionic, amphoteric, and/or zwitterionic surfactants can be included in the compositions of the present invention in addition to one or more anionic surfactants to help stabilize foam, to provide a suitable viscosity, or to give other functional or esthetic properties to the composition.

Usually, the anionic cleansing surfactant includes a hydrophobic moiety, such as a carbon chain including from about 8 carbon atoms to about 30 carbon atoms, and particularly from about 12 carbon atoms to about 20 carbon atoms, and further includes a hydrophilic moiety, such as a sulfate, sulfonate, carbonate, phosphate, or carboxylate. Often, the hydrophobic carbon chain is etherified, such as with ethylene oxide or propylene oxide, to impart a particular physical property, such as increased water-solubility or reduced surface tension, to the anionic cleansing surfactant.

Suitable anionic cleansing surfactants include, but are not limited to, compounds in the classes known as alkyl sulfates, alkyl ether sulfates, alkyl ether sulfonates, sulfate esters of an alkylphenoxy polyoxyethylene ethanol, alpha-olefin sulfonates, beta alkyloxy alkene sulfonates, alkyl arylsulfonates, alkyl carbonates, alkyl ether carboxylates, fatty acids, succinamates, sulfosuccinates, sarcosinates, octoxynol or nonoxynol phosphates, taurates, fatty taurides, sulfated monoglycerides, fatty acid amino polyoxyethylene sulfates, and isothienates, or combinations thereof. Many additional anionic cleansing surfactants are described in McCUTCHEON'S DETERGENTS AND EMULSIFIERS, 1989 ANNUAL, published by McCutcheon's Division MC Publishing Company, herein incorporated by reference. Usually, the anionic cleansing surfactant is present in the composition as a neutralized salt in the form of a sodium, potassium, lithium, ammonium, alkylammonium, or hydroxyalkylammonium salt, wherein the alkyl moiety includes from 1 to about 3 carbon atoms. The alkyl magnesium salts of lauryl sulfate, dodecylbenzenesulfonate, lauryl sulfosuccinate, lauryl ether sulfate, lauryl ether carboxylate, lauryl sarcosinate, cocomethyl tauride, and sulfosuccinate half ester amide, or combinations thereof. An especially useful anionic cleansing surfactant is a mixture of a lauryl sulfate salt and a lauryl ether sulfate salt.

It is well known that the inclusion of a substantial amount, e.g., 1.5% or more, of silicone conditioning agents in a conditioning shampoo substantially lowers the amount of foam generated by strong anionic detergents (e.g., alkyl sulfates, alkyl sulfonates, alkyl ether sulfates, and alkyl ether sulfonates). Accordingly, anionic detergents have been included in a higher percentage in shampoos containing silicone conditioning agents, and/or foam boosters have been added to the conditioning shampoos to provide the consumer with high foaming levels that most consumers perceive as essential to good cleansing. In addition to the decrease in foaming resulting from the inclusion of silicone and other conditioning agents present in a conditioning shampoo, suspending agents used to suspend the conditioning agent(s) also have detracted from the foaming levels achieved and sometimes have lowered the conditioning benefits imparted by the conditioning agent(s). Some of the suspending agents that lower foam levels and conditioning benefits include the acyl derivatives, amine oxides and the like, as disclosed in the Grote U.S. Patent No. 4,741,855

The conditioning shampoos of the present invention achieve excellent conditioning, foaming, and stability without the derivatives or amine oxides disclosed to be necessary for stability in Grote U.S. Patent No. 4,741,855. The ability to provide a conditioning shampoo that has excellent conditioning benefits, as well as excellent foaming and stability, has been a long-felt need in the conditioning shampoo art. The conditioning shampoos of the present invention solve this long-felt need by including a cationic polyethyleneimine in combination with a long-chain fatty alcohol to stabilize the suspension of silicone and other water-insoluble conditioning agents without substantially decreasing the foaming or conditioning benefits.

Surprisingly, protonated polyethyleneimine, together with the long chain alcohol, provides excellent stability while achieving additional conditioning benefits from the protonated polyethyleneimine. It should be understood that the polyethyleneimine can be added to the composition of the present invention in the protonated form, or can be protonated in situ (after addition to the composition) by an acid that is in the composition for pH control, and PEI protonation if needed. The molecular weight of the polyethyleneimine is not critical and can be any molecular weight commercially available, e.g., protonated polyethyleneimines available from BASF Corporation having a weight average molecular weight in the range of about 700 to about 70,000. The ability to provide a conditioning shampoo that has excellent conditioning benefits, as well as excellent foaming and stability, has been a long-felt need in the conditioning shampoo art. The conditioning shampoos of the present invention solve this long-felt need by including a protonated polyethyleneimine and a long-chain (C₈-C₁₈) fatty alcohol. The structure of the protonated polyethyleneimine is as follows:

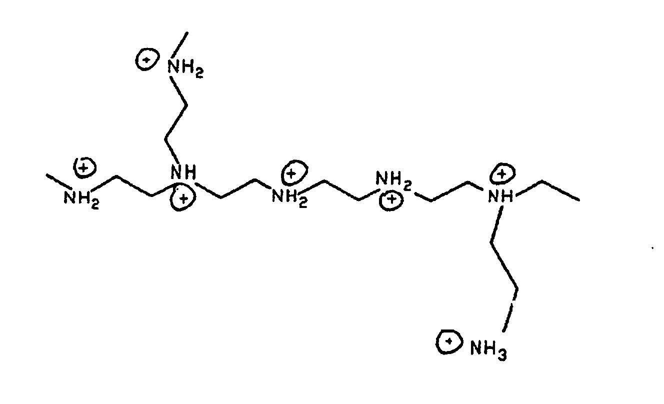

The preferred protonated polyethyleneimines have a ratio of primary: secondary: tertiary nitrogen atoms of about 1:2:1, respectively.

The fatty alcohol, in addition to the cationic polyethyleneimine, provides good stability to the composition while unexpectedly maintaining foaming and conditioning benefits. In accordance with a while maintaining the conditioning benefits imparted by one or more suspended, water-insoluble silicone conditioning agents.

Foam boosters can be added to the compositions of the present invention to further increase the foam volume attributed by the anionic and other detergents. Suitable foam boosters include, for example, polyvinylpyrrolidone (PVP); polyox; cetyl betaine; cocamide; cocamidoethyl betaine; cocamidopropyl betaine; cocamidopropyl hydroxysultaine; cocoamphodipropionic acid; coco-betaine; coco/oleamidopropyl betaine; coco-sultaine; cocoyl hydroxyethyl imidazoline; DEA-cocoamphodipropionate; DEA-lauraminopropionate; decyl betaine; disodium isostearyl sulfosuccinate; isopropyl stearate; lauramide; lauramidopropyl betaine; lauryl betaine; lauryl sultaine; myristamidopropyl betaine; myristaminopropionic acid; myristyl betaine; oleamidopropyl betaine; oleamidopropyl hydroxysultaine; oleyl betaine; palmamidopropyl betaine; palmitamidopropyl betaine; PEG-6 cocamide; PEG-3 lauramide; PEG-5 lauramide; PEG-6 lauramide; sodium cocoamphoacetate; sodium cocoamphopropionate; sodium lauraminopropionate; sodium lauroamphopropionate; sodium lauroyl sarcosinate; sodium myristoamphoacetate; sodium myristoyl sarcosinate; TEA-hydrogenated tallow glutamate; TEA-lauraminopropionate; TEA-myristaminopropionate; and undecylenamidopropylamine oxide.

To achieve the full advantage of the present invention, an optional nonionic alkanolamide is included in the conditioning shampoo composition in an amount of about 0.1% to about 5% by weight to provide sodium myristoyl sarcosinate; TEA-hydrogenated tallow glutamate; TEA-lauraminopropionate; TEA-myristaminopropionate; and undecylenamidopropylamine oxide.

To achieve the full advantage of the present invention, an optional nonionic alkanolamide is included in the conditioning shampoo composition in an amount of about 0.1% to about 5% by weight to provide exceptionally stable emulsification of water-insoluble conditioning agents and to aid in thickening and foam stability. Other useful suspending and thickening agents can be used instead of the alkanolamides, such as sodium alginate, guar gum, xanthan gum, gum arabic, cellulose derivatives, such as methylcellulose, hydroxybutylcellulose, hydroxyethylcellulose, hydroxypropylcellulose, and carboxymethylcellulose, and various synthetic polymeric thickeners, such as the polyacrylic acid derivatives. Suitable alkanolamides include, but are not limited to, those known in the art of hair care formulations, such as cocamide monoethanolamide (MEA), cocamide diethanolamide (DEA), soyamide DEA, lauramide DEA, oleamide monoisopropylamide (MIPA), stearamide MEA, myristamide MEA, lauramide MEA, capramide DEA, ricinoleamide DEA, myristamide DEA, stearamide DEA, oleylamide DEA, tallowamide DEA, lauramide MIPA, tallowamide MEA, isosteramide DEA, isostearamide MEA, and combinations thereof.

Fatty alcohols useful in the present formulations are primary or secondary alcohols having 8 to 18 carbons, inclusive, either as single long-chain lengths (e.g., 8, 10, 12, 14, 16, or 18 carbon atoms in length) or as a mixture of long-chain lengths of from 8 to 18 carbons, inclusive, are preferred. Several such mixtures are commercially available and are exemplified by mixtures of such alcohols (including ethoxylated alcohols of the same chain length range), such as ALFONIC 1216 from Vista Chemical Corporation. to about 3, and preferably, an average of 1 or 2 moles Further, fatty alcohols of other chain lengths can be used in addition to the C₈ to C₁₈ fatty alcohols so long as the C₈-C₁₈ alcohols are predominant, and present in an amount of 2 to 10% based on the total weight of the composition, preferably 2 to 5 weight percent.

The composition of the present invention also includes from about 0.1% to about 10%, and preferably from about 1.0% to about 5.0%, by weight of a non-volatile silicone compound. The preferred non-volatile silicone compound is a polydimethylsiloxane compound, such as a mixture, in about a 2:1 ratio, of a lower molecular weight polydimethysiloxane fluid (oil) and a higher molecular weight polydimethylsiloxane gum. The non-volatile polydimethylsiloxane compound is added to the composition of the present invention in an amount sufficient to provide improved combing and improved feel (softness) to the hair after shampooing. As referred to herein, silicone fluids and gums are those nonfunctional siloxanes having a viscosity of from about 5 to about 600,000 centistokes at 25°C. The so-called rigid silicones, as described in U.S. Patent No. 4,902,499, herein incorporated by reference, having non-volatile polydimethylsiloxane compound is added to the composition of the present invention in an amount sufficient to provide improved combing and improved feel (softness) to the hair after shampooing. As referred to herein, silicone fluids and gums are those nonfunctional siloxanes having a viscosity of from about 5 to about 600,000 centistokes at 25°C. The so-called rigid silicones, as described in U.S. Patent No. 4,902,499, herein incorporated by reference, having a viscosity above 600,000 centistokes at 20°C, e.g., 700,000 centistokes plus and a weight average molecular weight of at least about 500,000 also are useful in accordance with the present invention.

Preferred silicone gums include linear and branched polydimethyl siloxanes of the following general formula:

(CH₃)₃SiO--[Si(CH₃)₂)]ₙ--Si(CH₃)₃,

wherein n is from about 2,000 to about 15,000, preferably from about 2,000 to about 7,000. Silicone gums useful in compositions of the present invention are available from a variety of commercial sources, including General Electric Company and Dow Corning.

To achieve the best stability, the water, the anionic surfactant(s) and the cationic polyethyleneimine are mixed first and heated to a temperature above the melting point of the fatty alcohol, e.g., 150-200°F. The fatty alcohol then is added until completely dissolved in the water. The silicone or silicone blend then can be added and the mixture is agitated vigorously to shear and break up the silicone material into droplets preferably to a size less than about 10 micrometers, and more preferably to a size of about 5 microns to about 100 microns. The mixture is maintained at a temperature above the melting point of the fatty alcohol until all components are added.

To achieve the best results at the point that the silicone is sheared, the composition should have a viscosity in the range of about 5,000 to about 20,000 centipoises so that upon vigorous mixing or shearing, the resulting silicone droplets have a particle size of about 5 microns to about 100 microns, and preferably about 10 microns to about 30 microns, and are stable in the oil phase. As an example of the vigorous mixing, a six-bladed axial flow turbine impeller rotating at a speed of about 500 to 800 r.p.m. provides sufficient shearing of the non-volatile silicone material resulting in silicone droplets within the size range of about 5 to about 100 microns, and the sheared silicone droplets are exceptionally stable. The shearing of the silicone within a viscous composition provides the best silicone particle size and stability to the composition. If the particle size of the silicone droplets is less than about 5 microns, the silicone surface area is too great resulting in reduced foam. If the particle size of the silicone droplets is greater than about 100 microns, the silicone droplets have a tendency to coalesce and separate from the composition.

Other common cosmetic components and additives can be included in the compositions of the present invention, as long as the basic properties of the hair shampoos and shampoo conditioners are not adversely affected. Such optional cosmetic components and additives include, but are not limited to, nonionic surfactants, amphoteric surfactants, fragrances, dyes, hair colorants, opacifiers, pearlescing agents, thickeners, dandruff-control agents, hydrotropes, foam stabilizers, solubilizers, preservatives, water-softening agents, acids, alkalis, buffers, and the like. These optional components and additives usually are present in weight percentages of less than about 5% by weight each, and usually from about 0.1% to about 20% by weight of the composition in total.

For example, to improve consumer acceptance, both skin mildness and enhanced composition esthetics can be achieved by optionally including polyvinyl pyrrolidone and/or an amphoteric surfactant in the hair shampoo/conditioner in an amount ranging from 0% to about 5% by weight of the composition.

Suitable amphoteric surfactants that can be included in the compositions of the present invention include, but are not limited to, betaines, and hydroxypropylsultaines, or combinations thereof. Examples of amphoteric surfactants include, but are not limited to, cocamidopropyl betaine, lauramidopropyl betaine, coco/oleamidopropyl betaine, coco betaine, oleyl betaine, cocamidopropyl hydroxysultaine, tallow amidopropyl hydroxysultaine and dihydroxyethyl tallow glycinate, or combinations thereof. In general, any amphoteric surfactant can be included in the composition of the present invention as long as the stability, the conditioning, and the cleansing efficiency of the composition are not adversely affected.

The hair shampoo/conditioner compositions of the present invention also can include nonionic surfactants to help impart esthetic, physical, or cleansing properties to the composition. Likewise, the compositions can include other emulsifiers, conditioning agents, inorganic salts, humectants, and similar materials to provide the composition with desirable esthetic or physical properties. Generally, such optional ingredients are present in weight percentages of from about 0% to about 5% each, and from about 0% to about 20% in total, relative to the total weight of the composition.

The carrier of the hair shampoo/conditioner composition of the present invention is predominantly water, but non-aqueous solvents also can be included to help solubilize composition ingredients that are not sufficiently soluble in water, to adjust the viscosity of the composition or to act as a humectant. Suitable solvents include polyols, such as glycerol; glycols, such as ethylene glycol, propylene glycol and hexylene glycol, or mixtures thereof. The optional non-aqueous solvents should not adversely affect the ability of the composition to cleanse and condition the hair or adversely affect consumer appeal of the composition. A non-aqueous solvent can be present in the hair shampoo/conditioner composition of the present invention in an amount ranging from about 0% to about 5% by weight of the composition.

To achieve the full advantage of the present invention, the hair shampoo/conditioner composition is a relatively viscous mixture, e.g., about 3000 to about 8000 centipoises, that is stable indefinitely at temperatures normally found in commercial product storage and shipping. The compositions also have demonstrated sufficient stability to phase separation or precipitation of ingredients at temperatures normally found in commercial product storage and shipping to remain unaffected for periods of one year or more.

The following examples illustrate conditioning shampoos made in accordance with the present invention. It should be understood that the significant figures presented in the example data are not necessarily accurate to three or four significant figures, but, at times, are presented as such to attempt to accurately represent the very small quantities of the dyes included in the compositions, with water quantities calculated after taking into account the small dye quantities.

**EXAMPLE 1**

| Item # | Description | Actual Wt. % |
|---|---|---|
| 1 | DEIONIZED WATER | 30.761 |
| 2 | POLYETHYLENEIMINE* (50%) | 0.100 |
| 3 | AMMONIUM LAURETH SULFATE (2 MOLES OF ETHTOXYLATION) (30% ACTIVE) | 10.000 |
| 4 | AMMONIUM LAURYL SULFATE, (30% ACTIVE) | 10.000 |
| 5 | POLYVINYLPYRROLIDONE | 0.500 |
| 6 | DISTEARYLDIMONIUM CHLORIDE | 0.300 |
| 7 | TETRASODIUM EDTA, (39% ACTIVE) | 0.200 |
| 8 | AMMONIUM LAURYL SULFATE, (15% ACTIVE) | 40.000 |
| 9 | COCAMIDE MEA | 1.000 |
| 10 | (C₁₂-C₄₀ ALCOHOLS) | 2.000 |
| 11 | CITRIC ACID | 0.500 |
| 12 | LAURYL BETAINE, (40% ACTIVE) | 2.000 |
| 13 | D&C YELLOW #10 (1%) | 0.080 |
| 14 | FD&C BLUE #1 (1%) | 0.009 |
| 15 | SILICONE BLEND (33% GUM/67% OIL) | 2.000 |
| 16 | FRAGRANCE | 0.500 |
| 17 | METHYLCHLOROISOTHIAZOLINONE/METHYLISOTHIAZOLINONE | 0.050 |

| | | |
|---|---|---|
| * Polymin P (BASF Corporation) | | |

### Mixing Procedure Example 1:

Add item #1 into the compounding tank.

Add item #2, mix until dissolve.

Add items #3, #4 and #5, begin heating to 190-195°F.

Be careful of the excessive steam being released.

Turn agitation on high and add item #6, mix until completely dispersed.

Add item #7, mix well.

Add item #8, mix well.

Add item #9.

Add item #10, mix until completely dissolved.

Add item #11, continue heating to 190-195°F.

Maintain temperature at 190-195°F for 1 hour while mixing at high speed.

Check to make certain that the batch is completely melted and uniform, then add item #12, begin cooling at a rate of 1°F/minute using moderate agitation.

Dilute #13 and #14 in water, then add to batch.

At 110°F, increase agitation to high, (do not aerate) and add item #15.

CONTINUE COOLING AND MIXING AT HIGH SPEED. AVOID AERATION.

Add item #16 and #17.

**EXAMPLE 2**

| Item # | Description | Actual Wt. % |
|---|---|---|
| 1 | DEIONIZED WATER | 29.11 |
| 2 | POLYETHYLENEIMINE* (50%) (X-LINKED WITH 1,2 DICHLOROETHANE) | 0.20 |
| 3 | POLYVINYLPYRROLIDONE | 0.50 |
| 4 | AMMONIUM LAURETH SULFATE (2 MOLES OF ETHOXYLATION) (30% ACTIVE) | 10.00 |
| 5 | AMMONIUM LAURYL SULFATE, (15% ACTIVE) | 50.00 |
| 6 | DISTEARYLDIMONIUM CHLORIDE | 0.30 |
| 7 | COCAMIDE MEA | 1.25 |
| 8 | (C₁₂-C₄₀ ALCOHOLS) | 2.00 |
| 9 | BEHENIC ACID | 1.25 |
| 10 | LAURYL BETAINE, (40% ACTIVE) | 2.00 |
| 11 | SILICONE BLEND (33% GUM/67% OIL) | 2.50 |
| 12 | FRAGRANCE | 0.50 |
| 13 | METHYLCHLOROISOTHIAZOLINONE/METHYLISOTHIAZOLINONE (PRESERVATIVE) | 0.08 |
| 14 | TETRADOSIUM EDTA, (39% ACTIVE) (PRESERVATIVE) | 0.10 |
| 15 | POLYSORBATE 20 (PRESERVATIVE) | 0.15 |
| 16 | D&C YELLOW #10 (1%) (COLOR) | 0.04 |
| 17 | FD&C BLUE #1 (1%) (COLOR) | 0.02 |

| | | |
|---|---|---|
| * Polymin PS (BASE Corporation) | | |

### Mixing Procedure Example 2:

Add #1 into compounding tank.

Add #2, mix with high agitation.

Add #3, #4 and #5; mix well.

Begin heating to 180-185°F.

Add #6, mix until all of #5 is dispersed.

Add #7; mix well.

Add #8, mix until completely dissolved.

Add #9. Continue heating batch to 180-185°F. Maintain temperature at 180-185°F for one hour while mixing at high speed. Check to make certain that the batch is completely melted and uniform, then add #10, and begin cooling using moderate agitation.

Add #16 and #17 diluted 1:10 with deionized water.

Continue cooling the batch. At 110°F, increase agitation to high. DO NOT AERATE.

Add #11. Mix until all of #10 is dispersed and uniform with batch.

Add #12 and #13; mix well.

Add #14 and #15, mixing well after each addition.

**EXAMPLE 3**

| Item # | Description | Actual Wt. % |
|---|---|---|
| 1 | SOFT WATER | 27.3294 |
| 2 | POLYETHYLENEIMINE 50% (X-LINKED WITH 1,2 DICHLOROETHANE) | 3.0000 |
| 3 | SODIUM LAURYL SULFATE (DYNOL 120, 30% ACTIVE) | 27.5000 |
| 4 | COCAMIDE MEA | 2.0000 |
| 5 | LAURYL ALCOHOL | 2.0000 |
| 6 | SILICONE BLEND (33% GUM/67% OIL) | 3.0000 |
| 7 | DISTEARYLDIMONIUM CHLORIDE | 1.5000 |
| 8 | AMMONIUM LAURYL SULFATE, 30% | 27.5000 |
| 9 | ISODECYL LAURATE (EMOLLIENT) | 2.0000 |
| 10 | LIQUID CITRIC ACID 50% (PH AND PROTONATION OF PEI) | 3.2500 |
| 11 | FD&C GREEN #3 (10%) | 0.0006 |
| 12 | D&C YELLOW #10 (1%) | 0.1700 |
| 13 | FRAGRANCE PA 70258 | 0.6000 |
| 14 | METHYL & METHYLCHLORO ISOTHIAZOLINONE/K (PRESERVATIVE) | 0.0500 |
| 15 | DMDM HYDANTOIN/GLYDANT (PRESERVATIVE) | 0.1000 |

### Mixing Procedure Example 3:

Add the soft water (#1) to compounding tank.

Add SLS (#3) to the agitation on moderate.
Begin heating to 180-185°F.

Add the polyethyleneimine (#2) to the container.

At 150°F add cocamide MEA (#4)
Mix until melted.

At 160°F add lauryl alcohol (#5).
Mix until homogeneous.

At 180°F add silicone blend
with slightly higher agitation.
Mix for 15 minutes.

Add arosurf TA-100 with high agitation.
Mix for 60 minutes at 180-185°F.

Begin cooling and add ALS at a slow rate
over a course of 5 minutes.
Lower agitation to moderate.

Add isodecyl laurate.
Mix until uniform.

Add citric acid 50%.
Mix for 5 minutes.

At 110°F add remaining ingredients.
Mix until uniform.

| Specifications: | |
|---|---|
| | |
| 1. Property:- | pH measurements |
| Specification Data: | 5.5 - 6 |
| | (5.93 observed) |
| 2. Property: | T-SPINDLE VISCOSITY |
| Specification Data at 80°F: | 20 - 60 |
| 3. Property: | VISCOSITY |
| Specification Data at 80°F: | 400 - 7000 cps. (observed): pH = 5.93 Viscosity = 2000 cps. |
| Method of Analysis: | AP#5-0004.02D |

**EXAMPLE 4**

| Item # | Description | Actual Wt. % |
|---|---|---|
| 1 | SOFT WATER | 28.4794 |
| 2 | DYNOL 120 | 27.5000 |
| 3 | POLYETHYLENEIMINE 50% (X-LINKED WITH 1,2 DICHLOROETHANE) | 3.0000 |
| 4 | COCAMIDE MEA | 2.0000 |
| 5 | LAURYL ALCOHOL | 2.0000 |
| 6 | SILICONE BLEND (33% GUM/67% OIL) | 3.0000 |
| 7 | DISTEARYLDIMONIUM CHLORIDE | 1.5000 |
| 8 | AMMONIUM LAURYL SULFATE, 30% | 27.5000 |
| 9 | HYDROCHLORIC ACID 31% (PH AND PROTONATION OF PEI) | 2.1000 |
| 10 | ISODECYL LAURATE | 2.0000 |
| 11 | FD&C GREEN #3 (10%) | 0.0006 |
| 12 | D&C YELLOW #10 (1%) | 0.1700 |
| 13 | FRAGRANCE PA 70258 | 0.6000 |
| 14 | METHYL & METHYLCHLORO ISOTHIAZOLINONE/K (PRESERVATIVE) | 0.0500 |
| 15 | DMDM HYDANTOIN/GLYDANT (PRESERVATIVE) | 0.1000 |

### Mixing Procedure Example 4:

Add the soft water (#1) to a compounding tank.

Add sodiumlauryl sulfate (#2) to the tank.
Agitation on moderate.

Add the polyethyleneimine (#3) to the tank.
Mix well.
Begin heating to 180-185°F.

At 150°F add cocamide MEA (#4).
Mix until melted.

At 160°F add lauryl alcohol (#5).
Mix until homogeneous.

At 180°F add silicone blend (#6)
with slightly higher agitation.
Mix for 15 minutes.

Add arosurf TA-100 (#7) with high agitation.
Mix for 60 minutes at 180-185°F.

Begin cooling and add the hydrochloric acid 31% (#9).
Mix well.

Add the ALS (#8) at a slow rate
over a course of 5 minutes.
Lower agitation to moderate.

Add isodecyl laurate (#10).
Mix until uniform.

At 110°F add remaining ingredients.
Mix until uniform.

### Specification:

pH = 5.1

The following Example 5 shows that the combination of PEI with a long-chain (C₈-C₁₈) fatty alcohol provides stability without the alkanolamide (cocamide MEA) of the previous Examples:

### EXAMPLE 5

| Item # | Description | Actual Wt. % |
|---|---|---|
| 1 | SOFT WATER | 28.5794 |
| 2 | DYNOL 120 | 27.5000 |
| 3 | POLYETHYLENEIMINE 50% (X-LINKED WITH 1,2 DICHLOROETHANE) | 3.0000 |
| 4 | LAURYL ALCOHOL | 2.0000 |
| 5 | SILICONE BLEND (33% GUM/67% OIL) | 3.0000 |
| 6 | DISTEARYLDIMONIUM CHLORIDE | 1.5000 |
| 7 | AMMONIUM LAURYL SULFATE, 30% | 27.5000 |
| 8 | LIQUID CITRIC ACID 50% | 4.0000 |
| 9 | ISODECYL LAURATE | 2.0000 |
| 10 | FD&C GREEN #3 (10%) | 0.0006 |
| 11 | D&C YELLOW #10 (1%) | 0.1700 |
| 12 | FRAGRANCE PA 70258 | 0.6000 |
| 13 | METHYL & METHYLCHLORO ISOTHIAZOLINONE/K (PRESERVATIVE) | 0.0500 |
| 14 | DMDM HYDANTOIN/GLYDANT (PRESERVATIVE) | 0.1000 |

### Mixing Procedure Example 5:

Add the soft water (#1) to a compounding tank.

Add SLS (#) to tank.
Agitation on moderate.

Add the polyethyleneimine (#3) to the tank. Mix well.
Begin heating to 180-185°F.

At 160°F add lauryl alcohol (#4).
Mix until homogeneous.

At 180°F add silicone blend (#5)
with slightly higher agitation.
Mix for 15 minutes.

Add arosurf TA-100 (#6) with high agitation.
Mix for 60 minutes at 180-185°F.

Begin cooling and add the citric acid 50% (#8).
Mix well.

Add the ALS (#7) at a slow rate
over a course of 5 minutes.
Lower agitation to moderate.

Add isodecyl laurate (#9).
Mix until uniform.

At 110°F add remaining ingredients.
Mix until uniform.

The composition of Example 6 (without the fatty alcohol) precipitated upon addition of ammonium lauryl sulfate (#7) and could not be stabilized.

### EXAMPLE 6( not according to the invention)

| Item # | Description | Actual Wt. % |
|---|---|---|
| 1 | SOFT WATER | 28.5794 |
| 2 | DYNOL 120 | 27.5000 |
| 3 | POLYETHYLENEIMINE 50% (X-LINKED WITH 1,2 DICHLOROETHANE) | 3.0000 |
| 4 | COCAMIDE MEA | 2.0000 |
| 5 | SILICONE BLEND (33% GUM/67% OIL) | 3.0000 |
| 6 | DISTEARYLDIMONIUM CHLORIDE | 1.5000 |
| 7 | AMMONIUM LAURYL SULFATE, 30% | 27.5000 |
| 8 | LIQUID CITRIC ACID 50% | 4.0000 |
| 9 | ISODECYL LAURATE | 2.0000 |
| 10 | FD&C GREEN #3 (10%) | 0.0006 |
| 11 | D&C YELLOW #10 (1%) | 0.1700 |
| 12 | FRAGRANCE PA 70258 | 0.6000 |
| 13 | METHYL & METHYLCHLORO ISOTHIAZOLINONE/K (PRESERVATIVE) | 0.05000 |
| 14 | DMDM HYDANTOIN/GLYDANT (PRESERVATIVE) | 0.1000 |

### Mixing Procedure Example 6:

Add the soft water (#1) to a compounding tank.

Add SLS (#2) to tank.
Agitation on moderate.

Add the polyethyleneimine (#3) to the tank.
Mix well.
Begin heating to 180-180°F.

At 150°F add cocamide MEA (#4).
Mix until melted.

At 180°F add silicone blend (#5) with
slightly higher agitation.
Mix for 15 minutes.

Add arosurf TA-100 (#6)
with high agitation.
Mix for 60 minutes at 180-185°F.

Begin cooling and add the citric acid 50% (#8).
Mix well.

Add the ALS (#7) at a slow rate
over a course of 5 minutes.
Lower agitation to moderate.

Add isodecyl laurate (#9).
Mix until uniform.

At 110°F add remaining ingredients.
Mix until uniform.

The following composition remained stable for one week at 110°F without the di-long chain quaternary ammonium compound (distearyldimonium chloride) of the previous Examples:

### EXAMPLE 7

| Item # | Description | Actual Wt. % |
|---|---|---|
| 1 | SOFT WATER | 28.0794 |
| 2 | DYNOL 120 | 27.5000 |
| 3 | POLYETHYLENEIMINE 50% (X-LINKED WITH 1,2 DICHLOROETHANE) | 3.0000 |
| 4 | COCAMIDE MEA | 2.0000 |
| 5 | LAURYL ALCOHOL | 2.0000 |
| 6 | SILICONE BLEND (33% GUM/67% OIL) | 3.0000 |
| 7 | DYNOL ALS | 27.5000 |
| 8 | LIQUID CITRIC ACID 50% | 4.0000 |
| 9 | ISODECYL LAURATE | 2.0000 |
| 10 | FD&C GREEN #3 (10%) | 0.0006 |
| 11 | D&C YELLOW #10 (1%) | 0.1700 |
| 12 | FRAGRANCE PA 70258 | 0.6000 |
| 13 | KATHON CG | 0.0500 |
| 14 | DMDM HYDANTOIN/GLYDANT (PRESERVATIVE) | 0.1000 |

### Mixing Procedure Example 7:

Add the soft water (#1) to a compounding tank.

Add SLS (#2) to the tank.
Agitation on moderate.

Add the polyethyleneimine (#3) to the tank.
Mix well.
Begin heating to 180-185°F.

At 150°F add cocamide MEA (#4).
Mix until melted.

At 160°F add lauryl alcohol (#5).
Mix until homogeneous.

At 180°F add silicone blend (#6) with
slightly higher agitation.
Mix for 30 minutes at 180-185°F.

Begin cooling and add the citric acid 50% (#8).
Mix well.

Add the ALS (#7) at a slow rate over
a course of 5 minutes.
Lower agitation to moderate.

Add isodecyl laurate (#9).
Mix until uniform.

At 110°F add remaining ingredients.
Mix until uniform.

The following composition remained stable without the isodecyl laurate of the previous Examples:

### EXAMPLE 8

| Item # | Description | Actual Wt. % |
|---|---|---|
| 1 | SOFT WATER | 28.5794 |
| 2 | DYNOL 120 | 27.5000 |
| 3 | POLYETHYLENEIMINE 50% (X-LINKED WITH 1,2 DICHLOROETHANE) | 3.0000 |
| 4 | COCAMIDE MEA | 2.0000 |
| 5 | LAURYL ALCOHOL | 2.0000 |
| 6 | SILICONE BLEND (33% GUM/67% OIL) | 3.0000 |
| 7 | DISTEARYLDIMONIUM CHLORIDE | 1.5000 |
| 8 | AMMONIUM LAURYL SULFATE, 30% | 27.5000 |
| 9 | LIQUID CITRIC ACID 50% | 4.0000 |
| 10 | FD&C GREEN #3 (10%) | 0.0006 |
| 11 | D&C YELLOW #10 (1%) | 0.1700 |
| 12 | FRAGRANCE PA 70258 | 0.6000 |
| 13 | METHYL & METHYLCHLORO ISOTHIAZOLINONE/K (PRESERVATIVE) | 0.0500 |
| 14 | DMDM HYDANTOIN/GLYDANT (PRESERVATIVE) | 0.1000 |

### Mixing Procedure Example 8:

Add the soft water (#1) to a compounding tank.

Add SLS (#2) to the tank.
Agitation on moderate.

Add the polyethyleneimine (#3) to the tank.
Mix well.
Begin heating to 180-185°F.

At 150°F add cocamide MEA (#4).
Mix until melted.

At 160°F add lauryl alcohol (#5).
Mix until homogeneous.

At 180°F add silicone blend (#6) with
slightly higher agitation.
Mix for 15 minutes.

Add arosurf TA-100 (#7) with high agitation.
Mix for 60 minutes at 180-185°F.

Begin cooling and add the citric acid 50% (#9).
Mix well.

Add the ALS (#10) at a slow rate over
a course of 5 minutes.
Lower agitation to moderate.

At 110°F add remaining ingredients.
Mix until uniform.

The following composition remained stable without the alkanolamide (cocamide MEA) and without the di-long chain quaternary ammonium compound (distearyldimonium chloride) of the previous Examples:

### EXAMPLE 9

| Item # | Description | Actual Wt. % |
|---|---|---|
| 1 | DYNOL 120 | 27.5000 |
| 2 | POLYETHYLENEIMINE 50% (X-LINKED WITH 1,2 DICHLOROETHANE) | 3.0000 |
| 3 | LAURYL ALCOHOL | 2.0000 |
| 4 | SILICONE BLEND (33% GUM/67% OIL) | 3.0000 |
| 5 | AMMONIUM LAURYL SULFATE, 30% | 27.5000 |
| 6 | LIQUID CITRIC ACID 50% | 4.0000 |
| 7 | FD&C GREEN #3 (10%) | 0.0006 |
| 8 | ISODECYL LAURATE | 2.0000 |
| 9 | D&C YELLOW #10 (1%) | 0.1700 |
| 10 | FRAGRANCE PA 70258 | 0.6000 |
| 11 | METHYL & METHYLCHLORO ISOTHIAZOLINONE/K (PRESERVATIVE) | 0.0500 |
| 12 | DMDM HYDANTOIN/GLYDANT (PRESERVATIVE) | 0.1000 |
| 13 | SOFT WATER | 30.0794 |

### Mixing Procedure Example 9:

Add the soft water (#13) to a compounding tank.

Add SLS (#1) to the tank.
Moderate agitation.

Add PEI (#2) to the tank.
Mix well.

Add the lauryl alcohol (#3).

Add the silicone blend (#4).
Mix with high agitation for 30 minutes.

Add the ALS (#5).

Add the citric acid 50% (#6).

Add isodecyl laurate (#8).
Mix until uniform.

Add the dyes (#7), (#9) and (#11).

Add the remaining ingredients one at a time.
Mix until uniform.

### Comments:

After adding the ALS there was some precipitation. Heated batch up and precipitate gradually dissolved. Will repeat batch with initial heating.

The composition of Example 10 was prepared with initial heating prior to the addition of the ammonium lauryl sulfate to eliminate precipitation.

### EXAMPLE 10

| Item # | Description | Actual Wt. % |
|---|---|---|
| 1 | SOFT WATER | 32.0794 |
| 2 | DYNOL 120 | 27.5000 |
| 3 | POLYETHYLENEIMINE 50% (X-LINKED WITH 1,2 DICHLOROETHANE) | 3.0000 |
| 4 | LAURYL ALCOHOL | 2.0000 |
| 5 | SILICONE BLEND (33% GUM/67% OIL) | 3.0000 |
| 6 | AMMONIUM LAURYL SULFATE, 30% | 27.5000 |
| 7 | LIQUID CITRIC ACID 50% | 4.0000 |
| 8 | FD&C GREEN #3 (10%) | 0.0006 |
| 9 | D&C YELLOW #10 (1%) | 0.1700 |
| 10 | FRAGRANCE PA 70258 | 0.6000 |
| 11 | METHYL & METHYLCHLORO ISOTHIAZOLINONE/K (PRESERVATIVE) | 0.0500 |
| 12 | DMDM HYDANTOIN/GLYDANT (PRESERVATIVE) | 0.1000 |

### Mixing Procedure Example 10:

Add the soft water (#1) to a compound in the tank.

Add the SLS (#2).
Moderate agitation.
Begin heating to 160°F.

Add the PEI (#3).
Mix well.

Add the lauryl alcohol (#4).

Add the silicone blend (#5).
Mix with high agitation for 30 minutes.

Add the ALS (#6).

Add the citric acid 50% (#7).

Add the dyes (#8 and #9).

Add the remaining ingredients one at a time.
Mix until uniform.

### Comments:

After the lauryl alcohol addition (110°F) there was a slight precipitation that disappeared after a second. Finished batch is stable, opaque and uniform.

The following Example 11 shows that the composition of the present invention is stable, with silicone conditioning agents, without precipitation, without the alkanolamide (cocamide MEA), without the di-long chain quaternary ammonium compound (distearyldimonium chloride), and without the isodecyl laurate of the previous Examples:

### EXAMPLE 11

| Item # | Description | Actual Wt. % |
|---|---|---|
| 1 | SOFT WATER | 58.10 |
| 2 | POLYETHYLENEIMINE 50% (X-LINKED WITH 1,2 DICHLOROETHANE) | 1.50 |
| 3 | DYNOL 120 | 17.50 |
| 4 | LAURYL ALCOHOL | 2.00 |
| 5 | SILICONE BLEND (33% GUM/67% OIL) | 2.00 |
| 6 | DYNOL ALS | 17.50 |
| 7 | LIQUID CITRIC ACID 50% | 1.25 |
| 8 | KATHON CG (PRESERVATIVE) | 0.05 |
| 9 | DMDM HYDANTOIN/GLYDANT (PRESERVATIVE) | 0.10 |

### Mixing Procedure Example 11:

Add the soft water (#1) to the tank.
Moderate agitation.

Add the PEI (#2).

Add the SLS (#3).
Begin heating to 170-180°F.

At 150°F add the lauryl alcohol (#4).
Continue heating.

At 170-180°F add the silicone blend (#5)
with high agitation.
After 30 minutes at 170-180°F start cooling.

Slowly add the ALS (#6).

Slowly add the citric acid 50% (#7).

At 110°F add the preservatives (#8 and #9).
Mix until uniform.

### Comments:

After addition of the citric acid there was some very slight initial precipitation that went away immediately. The finished batch was opaque, homogeneous and thin viscosity (∼ 100 - 200 cps.). After 24 hours there was no precipitation.

The following comparative Examples 12-16 were compositions prepared in an attempt to eliminate the long-chain alcohol from the composition, without success:

### EXAMPLE 12

| Item # | Description | Actual Wt. % |
|---|---|---|
| 1 | SOFT WATER | 63.10 |
| 2 | POLYETHYLENEIMINE 50% (X-LINKED WITH 1,2 DICHLOROETHANE) | 1.00 |
| 3 | DYNOL 120 | 33.40 |
| 4 | COCAMIDE MEA | 1.00 |
| 5 | LIQUID CITRIC ACID 50% | 1.35 |
| 6 | KATHON CG (PRESERVATIVE) | 0.05 |
| 7 | DMDM HYDANTOIN/GLYDANT (PRESERVATIVE) | 0.10 |

### Mixing Procedure Example 12:

Add the soft water (#1) to the tank.
Moderate agitation.

Add the PEI (#2).

Add the SLS (#3).
Begin heating to 160-170°F.

At 140°F add the cocamide MEA (#4).
Continue heating.
After 15 minutes at 160-170°F start cooling.

Add the citric acid 50% (#5).

At 110°F add the preservatives (#6 and #7).
Mix until uniform.

### Comments:

After the addition of citric acid 50% (#5) there was gross precipitation of the PEI (#2). After additional heating beyond 170°F the precipitate did not solubilize. pH of solution was ≈ 5.5.

**EXAMPLE 13**

| Item # | Description | Actual Wt. % |
|---|---|---|
| 1 | SOFT WATER | 63.10 |
| 2 | POLYETHYLENEIMINE 50% (X-LINKED WITH 1,2 DICHLOROETHANE) | 1.10 |
| 3 | DYNOL 120 | 33.40 |
| 4 | GLYCERINE USP | 1.00 |
| 5 | LIQUID CITRIC ACID 50% | 1.25 |
| 6 | KATHON CG (PRESERVATIVE) | 0.05 |
| 7 | DMDM HYDANTOIN/GLYDANT (PRESERVATIVE) | 0.10 |

### Mixing Procedure Example 13

Add the soft water (#1) to the tank.
Moderate agitation.

Add the PEI (#2).

Add the SLS (#3).
Begin heating to 160-170°F.

At 140°F add the glycerine USP (#4).
Continue heating.
After 15 minutes at 160-170°F start cooling.

Add the citric acid 50% (#5).

At 110°F add the preservatives (#6 and #7).
Mix until uniform.

### Comments:

After the addition of the citric acid 50% (#5) there was gross precipitation of the PEI (#2). After additional heating beyond 170°F the precipitate did not solubilize.

**EXAMPLE 14**

| Item # | Description | Actual Wt. % |
|---|---|---|
| 1 | SOFT WATER | 63.10 |
| 2 | POLYETHYLENEIMINE 50% (X-LINKED WITH 1,2 DICHLOROETHANE) | 1.10 |
| 3 | DYNOL 120 | 33.40 |
| 4 | POLYSORBATE 20 | 1.00 |
| 5 | LIQUID CITRIC ACID 50% | 1.25 |
| 6 | KATHON CG (PRESERVATIVE) | 0.05 |
| 7 | DMDM HYDANTOIN/GLYDANT (PRESERVATIVE) | 0.10 |

### Mixing Procedure Example 14:

Add the soft water (#1) to the tank.
Moderate agitation.

Add the PEI (#2).

Add the SLS (#3).
Begin heating to 140-150°F.

At 120°F add the polysorbate 20 (#4).
Continue heating.
After 15 minutes at 140-150°F start cooling.

Add the citric acid 50% (#5).

At 110°F add the preservatives.
Mix until uniform.

### Comments:

After addition of citric acid (#5) at 150°F there was gross precipitation. After additional heating up to 175°F, the precipitate did not solubilize.

**EXAMPLE 15**

| Item # | Description | Actual Wt. % |
|---|---|---|
| 1 | SOFT WATER | 60.10 |
| 2 | POLYETHYLENEIMINE 50% (X-LINKED WITH 1,2 DICHLOROETHANE) | 1.50 |
| 3 | DYNOL 120 | 17.50 |
| 4 | DYNOL ALS | 17.50 |
| 5 | SILICONE BLEND (33% GUM/67% OIL) | 2.00 |
| 6 | LIQUID CITRIC ACID 50% | 1.25 |
| 7 | KATHON CG (PRESERVATIVE) | 0.05 |
| 8 | DMDM HYDANTOIN/GLYDANT (PRESERVATIVE) | 0.10 |

### Mixing Procedure Example 15:

Add the soft water (#1) to the tank.
Moderate agitation.

Add the PEI (#2)

Add the SLS (#3).
Begin heating to 140-150°F.

At 140°F add the silicone blend (#5)
with high agitation.
Continue heating.
After 30 minutes at 160-170°F start cooling.

Add the citric acid 505 (#6) with moderate agitation.

At 110°F add the preservatives (#7 and #8).
Mix until uniform.

### Comments:

After addition of the citric acid 50% (#6) there was gross precipitation. After additional heating > 170°F the precipitate did not solubilize.

**EXAMPLE 16**

| Item # | Description | Actual Wt. % |
|---|---|---|
| 1 | SOFT WATER | 59.10 |
| 2 | POLYETHYLENEIMINE 50% (X-LINKED WITH 1,2 DICHLOROETHANE) | 1.50 |
| 3 | DYNOL 120 | 17.50 |
| 4 | CETYL/STEARYL ALCOHOL* | 1.00 |
| 5 | SILICONE BLEND (33% GUM/67% OIL) | 2.00 |
| 6 | DYNOL ALS | 17.50 |
| 7 | LIQUID CITRIC ACID 50% | 1.25 |
| 8 | KATHON CG (PRESERVATIVE) | 0.05 |
| 9 | DMDM HYDANTOIN/GLYDANT (PRESERVATIVE) | 0.10 |

| | | |
|---|---|---|
| * 60% cetyl and 25-30% stearyl alcohol. | | |

### Mixing Procedure Example 16:

Add the soft water (#1) to the tank.
Moderate agitation.

Add the PEI (#2).

Add the SLS (#3).
Begin heating to 180-190°F.

At 170°F add the cetyl/stearyl alcohol (#4).
Continue heating.

At 180°F add the silicone blend (#5)
with high agitation.
After 30 minutes at 180-190°F start cooling.

Slowly add the ALS (#6).

Slowly add the citric acid 50% (#7).

At 110°F add the preservatives (#8 and #().
Mix until uniform.

### Comments:

After addition of the citric acid 50% (#7) there was precipitation of the PEI (#2)

**EXAMPLE 17**

| Item # | Description | Actual Wt. % |
|---|---|---|
| 1 | SOFT WATER | 60.10 |
| 2 | POLYETHYLENEIMINE 50% (X-LINKED WITH 1,2 DICHLOROETHANE) | 1.50 |
| 3 | DYNOL 120 | 17.50 |
| 4 | LAURYL ALCOHOL | 2.00 |
| 5 | DYNOL ALS | 17.50 |
| 6 | LIQUID CITRIC ACID 50% | 1.25 |
| 7 | KATHON CG (PRESERVATIVE) | 0.05 |
| 8 | DMDM HYDANTOIN/GLYDANT (PRESERVATIVE) | 0.10 |

### Mixing Procedure Example 17:

Add the soft water (#1) to the tank.
Moderate agitation.

Add the PEI (#2).

Add the SLS (#3).
Begin heating to 160-170°F.

At 150°F add the lauryl alcohol (#4).
Continue heating.
After 15 minutes at 160-170°F start cooling.

Slowly add the ALS (#5).

Slowly add the citric acid 50% (#6).

At 110°F add the preservatives (#7 and #8).
Mix until uniform.

### Comments:

After addition of citric acid 50% there was no precipitate and batch was opaque, homogeneous and thin. After 24 hours, batch was still stable. After 48-72 hours there was some solution separation. Water was separating to the top. When mixed, however, the solution was homogeneous. Even after several days of sitting it could be re-mixed and there was no precipitate.

The following comparative Example 18 shows that elimination of the lauryl alcohol of Example 17, and substitution with isodecyl laurate, will not provide a stable composition:

**EXAMPLE 18**

| Item # | Description | Actual Wt. % |
|---|---|---|
| 1 | SOFT WATER | 60.10 |
| 2 | POLYETHYLENEIMINE 50% (X-LINKD WITH 1,2 DICHLOROETHANE) | 1.50 |
| 3 | DYNOL 120 | 17.50 |
| 4 | ISODECYL LAURATE | 2.00 |
| 5 | DYNOL ALS | 17.50 |
| 6 | LIQUID CITRIC ACID 50% | 1.25 |
| 7 | KATHON CG (PRESERVATIVE) | 0.05 |
| 8 | DMDM HYDANTOIN/GLYDANT (PRESERVATIVE) | 0.10 |

### Mixing Procedure Example 18:

Add the soft water (#1) to the tank.
Moderate agitation.

Add the PEI (#2).

Add the SLS (#3).
Begin heating to 160-170°F.

At 150°F add the isodecyl laurate (#4).
Continue heating.
After 15 minutes at 160-170°F start cooling.

Slowly add the ALS (#5).

Slowly add the citric acid 50% (#6).

At 110°F add the preservatives (#7 and #8).
Mix until uniform.

### Comments:

After addition of the citric acid 50% (#6) there was gross precipitation that did not disappear after continued heating and mixing.

**EXAMPLE 19**

| Item # | Description | Actual Wt. % |
|---|---|---|
| 1 | SOFT WATER | 52.60 |
| 2 | POLYETHYLENEIMINE 50% (X-LINKED WITH 1,2 DICHLOROETHANE) | 1.00 |
| 3 | DYNOL 120 | 17.50 |
| 4 | LAURYL ALCOHOL | 10.00 |
| 5 | DYNOL ALS | 17.50 |
| 6 | LIQUID CITRIC ACID 50% | 1.40 |

### Mixing Procedure Example 19:

Add soft water (#1) to tank.
Agitation on moderate.

Add the polymin P (#2) to the tank.
Mix until dissolved.

Add the SLS (#3).
Begin heating to 160-170°F.

At 150°F add the lauryl alcohol (#4).
Hold at 160 - 170°F for 15 minutes.
Stop heating and allow to begin cooling.

Slowly add the ALS (#5).
Mix well.

Slowly titrate in the citric acid 50% (#6).
Mix well.

### Comments:

After adding alcohol solution, the composition thickened. Neutralized to pH = 5.5 w/citric acid. Solution thinned out. Solution was without precipitate. Stable at room temperature.

**EXAMPLE 20**

| Item # | Description | Actual Wt. % |
|---|---|---|
| 1 | SOFT WATER | 58.00 |
| 2 | POLYMIN P | 1.50 |
| 3 | SLS, 30% | 17.50 |
| 4 | OCTANOL, C₈ | 2.00 |
| 5 | ALS, 30% | 17.50 |
| 6 | LIQUID CITRIC ACID 50% | 3.50 |

### Mixing Procedure Example 20 (C₈ fatty alcohol):

Add soft water (#1) to tank.
Agitation on moderate.

Add the Polymin P (#2) to the tank.
Mixed until dissolved.

Add the SLS (#3) to the tank.
Begin heating to 160-170°F.

At 150°F add the octanol (#4).
Hold at 160-170°F for 15 minutes.
Stop heating and allow to begin cooling.

Slowly add the ALS (#5).
Mix well.

Slowly titrate in the citric acid 50% (#6).
Mix well.

### Comments:

pH = 6.0. The solution was slightly opaque without any apparent precipitate. However, after an inspection with a watch glass, there were many very fine particles present. Although there were particles (very fine), the solution could have been used very easily. A mixture of C₈ alcohol with C₁₂ alcohol would result in no particles.

**EXAMPLE 21**

| Item # | Description | Actual Wt. % |
|---|---|---|
| 1 | SOFT WATER | 58.00 |
| 2 | POLYMIN P | 1.50 |
| 3 | SLS, 30% | 17.50 |
| 4 | DECANOL C₁₀ | 2.00 |
| 5 | ALS, 30% | 17.50 |
| 6 | LIQUID CITRIC ACID 50% | 3.50 |

### Mixing Procedure Example 21:

Add soft water (#1) to tank.
Agitation on moderate.

Add the polymin P (#2).
Mix until dissolved.

Add the SLS (#3).
Begin heating to 160-170°F.

At 150°F add the C₁₀ alcohol (#4).
Hold at 160-170°F for 15 minutes.
Stop heating and allow to begin cooling.

Slowly add the ALS (#5).
Mix well.

Slowly titrate in the citric acid 50% (#6).
Mix well.

### Comments:

After processing the pH = ∼ 5.5. The solution was slightly opaque without any apparent precipitate. However, after an inspection with a watch glass, there were many very fine particles present. A mix with C₁₂ alcohol would eliminate the fine particles.

**EXAMPLE 22**

| Item # | Description | Actual Wt. % |
|---|---|---|
| 1 | SOFT WATER | 58.00 |
| 2 | POLYETHYLENEIMINE 50% (X-LINKED WITH 1,2 DICHLOROETHANE) | 1.50 |
| 3 | DYNOL 120 | 17.50 |
| 4 | MYRISTYL ALCOHOL | 2.00 |
| 5 | DYNOL ALS | 17.50 |
| 6 | LIQUID CITRIC ACID 50% | 3.50 |

### Mixing Procedure Example 22:

Add soft water (#1) to tank.
Agitation on moderate.

Add the Polymin P (#2).
Mix until dissolved.

Add the SLS (#3).
Begin heating to 160-170°F.

At 150°F add the myristyl alcohol (#4).
Hold at 160-170°F for 15 minutes.
Stop heating and allow to begin cooling.

Slowly add the ALS (#5).
Mix well.

Slowly titrate in the citric acid 50% (#6).
Mix well.

### Comments:

pH = 6. The batch was a milky white emulsion without precipitation or particles. Stable with slight agitation once a week.

**EXAMPLE 23**

| Item # | Description | Actual Wt. % |
|---|---|---|
| 1 | SOFT WATER | 58.00 |
| 2 | POLYETHYLENEIMINE 50% (X-LINKED WITH 1,2 DICHLOROETHANE) | 1.50 |
| 3 | DYNOL 120 | 17.50 |
| 4 | CETYL ALCOHOL | 2.00 |
| 5 | DYNOL ALS | 17.50 |
| 6 | LIQUID CITRIC ACID 50% | 3.50 |

### Mixing Procedure Example 23:

Add soft water (#1) to tank.
Agitation on moderate.

Add the Polymin P (32).
Mix until dissolved.

Add the SLS (#3).
Begin heating to 160-170°F.

At 150°f add the cetyl alcohol (#4).
Hold at 170-180°F for 15 minutes.
Stop heating and allow to begin cooling.

Slowly add the ALS (#5).
Mix well.

Slowly titrate in the citric acid 50% (#6)
Mix well.

### Comments:

After processing pH = 6.00 and batch was stable without precipitation. Solution separated slightly but when slightly agitated was homogeneous again. Stable with slight agitation.

**EXAMPLE 24**

| Item # | Description | Actual Wt. % |
|---|---|---|
| 1 | SOFT WATER | 59.75 |
| 2 | POLYMIN P | 0.50 |
| 3 | SLS, 30% | 17.50 |
| 4 | MYRISTYL ALCOHOL | 2.67 |
| 5 | UNILIN 350 (PREDOMINANTLY C₃₀) | 0.33 |
| 6 | ALS, 30% | 17.50 |
| 7 | LIQUID CITRIC ACID 50% | 1.75 |

### Mixing Procedure Example 24:

Add soft water (#1) to tank.
Agitation on moderate.

Add the Polymin P (#2) to the tank.
Mix until dissolved.

Add the SLS (#3) to the tank.
Begin heating to 160-170°F

At 150°F add the myristyl alcohol (#4)
and Unilin 350 (#5).
Hold at 170-180°F for 15 minutes.
Stop heating and allow to begin cooling.

Slowly add the ALS (#6) to the tank.
Mix well.

Slowly titrate in the citric acid 50% (#7).
Mix well.

### Comments

After processing there were no particles. Batch stable with shaking once a week.

**EXAMPLE 25**

| Item # | Description | Actual Wt. % |
|---|---|---|
| 1 | SOFT WATER | 73.00 |
| 2 | POLYETHYLENEIMINE 50% (X-LINKED WITH 1,2 DICHLOROETHANE) | 8.00 |
| 3 | DYNOL 120 | 7.00 |
| 4 | LAURYL ALCOHOL | 3.00 |
| 5 | LIQUID CITRIC ACID 50% | 9.00 |

### Mixing Procedure Example 25:

Add soft water (#1) to the tank.
Agitation on moderate.

Add the Polymin P (#2) to the tank.
Mix until dissolved.

Add the SLS (#2) to the tank.
Begin heating to 160-170°F.

At 150°F add the lauryl alcohol (#4).
Hold at 160-170°F for 15 minutes.
Stop heating and allow to begin cooling.

Slowly titrate in the citric acid 50% (#5).
Mix well.

### Comments:

During neutralization batch is okay.

**EXAMPLE 26**

| Item # | Description | Actual Wt. % |
|---|---|---|
| 1 | SOFT WATER | 60.60 |
| 2 | POLYETHYLENEIMINE 50% (X-LINKED WITH 1,2 DICHLOROETHANE) | 1.50 |
| 3 | DYNOL 120 | 17.50 |
| 4 | LAURYL ALCOHOL | 2.00 |
| 5 | DYNOL ALS | 17.50 |
| 6 | PHOSPHORIC ACID, 85% | 0.90 |

### Mixinq Procedure Example 26:

Add the soft water (#1) to the tank.
Moderate agitation.

Add the PEI (#2).

Add the SLS (#3).
Begin heating to 160-170°F.

At 150°F add the lauryl alcohol (#4).
Continue heating.
After 15 minutes at 160-170°F start cooling.

Slowly add the ALS (#5).

Slowly add the phosphoric acid 88% (#6).
Mix until uniform.

### Comments:

Batch was opaque without precipitation or particles. Agitated periodically.

**EXAMPLE 27**

| Item # | Description | Actual Wt. % |
|---|---|---|
| 1 | SOFT WATER | 60.55 |
| 2 | POLYMIN P | 0.75 |
| 3 | SLS, 30% | 17.50 |
| 4 | MYRISTYL ALCOHOL | 2.67 |
| 5 | C₁₂/C₁₆ (1 MOLE E.O.) ALCOHOL ETHOXYLATE | 0.33 |
| 6 | ALS, 30% | 17.50 |
| 7 | LIQUID CITRIC ACID 50% | 0.70 |

### Mixing Procedure Example 27:

Add the soft water (#1) to the tank.
Moderate agitation.

Add the PEI (#2) the to tank.

Add the SLS (#3) to the tank.
Begin heating to 160-170°F.

At 150°F add the myristyl alcohol (#4)
and C₁₂, C₁₆, C₂₂ (1 mole e.o.) alcohol ethylate (#5).
Continue heating. After 15 minutes at 160-170°F
start cooling.

Slowly add the ALS (#6) to the tank.

Slowly add the citric acid 50% (#7) to the tank.
Mix until uniform.

### Comments:

After processing batch was stable without any precipitate or particles. Agitated periodically.

## Claims

1. A hair conditioning shampoo composition comprising an emulsion of water; about 5 to about 65 percent by weight of an anionic cleaning surfactant; from 2 to 10 weight percent of a long-chain fatty alcohol having predominantly 8 to 18 carbons in the long chain; and from about 0.01 to 4.0 weight percent of a cationic polyethyleneimine, said composition having a pH of 4 to 8.

2. The composition according to Claim 1, further including 0.1 to about 10% by weight silicone conditioning agent.

3. The composition according to Claims 1 or 2, wherein the polyethyleneimine is included in an amount of 0.01% to 1% by weight.

4. The composition according to anyone of Claims 1 to 3, wherein said long-chain fatty alcohol is present in an amount of 2 to 5 weight percent of said composition

5. The composition of anyone of Claims 2 to 4, wherein the silicone has a viscosity at 25°C of at least 5 centistokes and a boiling point at 760 mm Hg pressure and 25°C of at least 250°C.

6. The composition of anyone of Claims 1 to 5 wherein the anionic cleaning surfactant is selected from the group consisting of alkyl sulfates; alkyl sulfonates; alkyl ether sulfates and alkyl ether sulfonates.

7. The composition of anyone of Claims 1 to 6, wherein the cationic polyethyleneimine has a weight average molecular weight in the range of 700 to 70,000.

8. A method of shampooing and conditioning hair which comprises applying to said hair the composition of anyone of Claims 1 to 7.

9. A method of manufacturing the conditioning shampoo composition of anyone of Claims 1 to 7 comprising:
heating a mixture of water, an anionic surfactant and a cationic polyethyleneimine to a temperature above the melting point of a fatty alcohol;
adding the fatty alcohol to said mixture until said alcohol is completely melted to form an aqueous fatty alcohol and polyethyleneimine surfactant solution;
adding a silicone conditioning agent to said solution to form a two-phase composition; and
vigorously agitating the two-phase composition to break up the silicone into droplets and suspend said silicone droplets.

## Patentansprüche

1. Haar konditionierende Shampoozusammensetzung, umfassend eine Emulsion von Wasser; etwa 5 bis etwa 65 Gewichtsprozent eines anionischen Reinigungstensids; 2 bis 10 Gewichtsprozent eines langkettigen Fettalkohols mit vorwiegend 8 bis 18 Kohlenstoffatomen in der langen Kette; und 0,01 bis 4,0 Gewichtsprozent eines kationischen Polyethylenimins, wobei die Zusammensetzung einen pH-Wert von 4 bis 8 aufweist.

2. Zusammensetzung nach Anspruch 1, die weiterhin 0,1 bis 10 Gewichtsprozent Silikonkonditionierungsmittel einschließt.

3. Zusammensetzung nach Ansprüchen 1 oder 2, wobei das Polyethylenimin in einer Menge von 0,01% bis 1 Gewichtsprozent eingeschlossen ist.

4. Zusammensetzung nach einem der Ansprüche 1 bis 3, wobei der langkettige Fettalkohol in einer Menge von 2 bis 5 Gewichtsprozent der Zusammensetzung vorliegt.

5. Zusammensetzung nach einem der Ansprüche 2 bis 4, wobei das Silikon eine Viskosität bei 25°C von mindestens 5 Centistokes und einen Siedepunkt bei 760 mmHg Druck und 25°C von mindestens 250°C aufweist.

6. Zusammensetzung nach einem der Ansprüche 1 bis 5, wobei das anionische Reinigungstensid aus der Gruppe, bestehend aus Alkylsulfaten, Alkylsulfonaten, Alkylethersulfaten und Alkylethersulfonaten, ausgewählt ist.

7. Zusammensetzung nach einem der Ansprüche 1 bis 6, wobei das kationische Polyethylenimin ein gewichtsmittleres Molekulargewicht im Bereich von 700 bis 70 000 aufweist.

8. Verfahren zum Shampoonieren und Konditionieren von Haar, das Auftragen der Zusammensetzung nach einem der Ansprüche 1 bis 7 auf das Haar umfasst.

9. Verfahren zum Herstellen der konditionierenden Shampoozusammensetzung nach einem der Ansprüche 1 bis 7, umfassend:
Erhitzen eines Gemisches von Wasser, einem anionischen Tensid und einem kationischen Polyethylenimin auf eine Temperatur oberhalb des Schmelzpunktes eines Fettalkohols;
Zugeben des Fettalkohols zu dem Gemisch bis der Alkohol vollständig geschmolzen ist, zur Bildung einer wässrigen Fettalkohol- und Polyethylenimin-Tensidlösung;
Zugeben eines Silikonkonditionierungsmittels zu der Lösung zur Bildung einer Zwei-Phasen-Zusammensetzung; und
heftiges Rühren der Zwei-Phasen-Zusammensetzung zum Aufbrechen des Silikons in Tröpfchen und Suspendieren der Silikontröpfchen.

## Revendications

1. Composition de shampoing de conditionnement pour les cheveux comprenant une émulsion d'eau ; d'environ 5 à environ 65 pourcent en poids d'un tensioactif anionique nettoyant ; d'environ 2 à 10 pourcent d'un alcool gras à longue chaîne ayant de façon prédominante de 8 à 18 atomes de carbone dans la longue chaîne ; et de 0,01 à 4,0 pourcent en poids d'un polyéthylène imine cationique, ladite composition ayant un pH de 4 à 8.

2. Composition selon la revendication 1, incluant en outre de 0,1 à 10 % en poids d'un agent de conditionnement silicone.

3. Composition selon la revendication 1 ou 2, dans laquelle le polyéthylène imine est inclus dans une quantité de 0,01 % à 1 % en poids.

4. Composition selon l'une quelconque des revendications 1 à 3, dans laquelle ledit alcool gras à longue chaîne est présent dans une quantité de 2 à 5 pourcent en poids de ladite composition.

5. Composition selon l'une quelconque des revendications 2 à 4, dans laquelle la silicone a une viscosité à 25°C d'au moins 5 centistokes et un point d'ébullition à 760 mm de pression de mercure et à 25°C qui est d'au moins 250°C.

6. Composition selon l'une quelconque des revendications 1 à 5, dans laquelle le tensioactif anionique nettoyant est sélectionné à partir du groupe constitué des alkyle sulfates ; des alkyl sulfonates ; des alkyl éther sulfates et des alkyl éther sulfonates.

7. Composition selon l'une quelconque des revendications 1 à 6, dans laquelle le polyéthylène imine cationique a une masse moléculaire comprise dans la gamme allant de 700 à 70.000.

8. Procédé pour shampouiner et conditionner les cheveux comprenant le fait d'appliquer sur lesdits cheveux la composition selon l'une quelconque des revendications 1 à 7.

9. Procédé de fabrication d'une composition de shampoing de conditionnement selon l'une quelconque des revendications 1 à 7, comprenant :
le fait de chauffer un mélange d'eau, un tensioactif anionique et un polyéthylène imine cationique à une température supérieure au point d'ébullition de l'alcool gras ;
ajouter l'alcool gras audit mélange jusqu'à ce que ledit alcool soit complètement fondu pour former un alcool gras aqueux et une solution tensioactive de polyéthylène imine ;
ajouter un agent de conditionnement silicone à ladite solution pour former une composition en deux phases ; et
agiter vigoureusement la composition à deux phases pour décomposer la silicone en gouttelettes et mettre en suspension lesdites gouttelettes de silicone.
